# FASCICULE DE BREVET EUROPEEN

(11) **EP 4 085 958 B1**
(45) Date de publication et mention de la délivrance du brevet: **15.11.2023**
(21) Numéro de dépôt: 22160682.5
(22) Date de dépôt: 08.03.2022
(51) Int. Cl.: A61M 16/06, A61M 16/12, A61M 16/20

(54) **DISPOSITIF DE VENTILATION À PRESSION CONTINUE ADAPTÉ AU TRAITEMENT DES PATIENTS EN INSUFFISANCE RESPIRATOIRE**
GERÄT ZUR KONTINUIERLICHEN DRUCKBEATMUNG, DAS FÜR DIE BEHANDLUNG VON PATIENTEN MIT ATEMINSUFFIZIENZ GEEIGNET IST
VENTILATION DEVICE WITH CONTINUOUS PRESSURE ADAPTED TO THE TREATMENT OF PATIENTS WITH RESPIRATORY FAILURE

(30) Priorité: 06.05.2021 FR 2104790
(43) Date de publication de la demande: 09.11.2022
(73) Titulaire: Air Liquide Medical Systems, 92182 Antony Cedex (FR); Air Liquide Medical Systems S.R.L., 20158 Milano (IT)
(72) Inventeur: BADAT, Bilal, 92182 Antony Cedex (FR); RICHARD, Jean-Christophe, 92182 Antony Cedex (FR); LESIMPLE, Arnaud, 92182 Antony Cedex (FR); PROUVEZ, Nathan, 92182 Antony Cedex (FR); MASSARO, Paolo, 25073 Bovezzo (IT); ZADRA, Davide, 25073 Bovezzo (IT); ALBERICI, Luca, 25073 Bovezzo (IT)
(74) Mandataire: Air Liquide

(56) Documents cités:
- EP-A1- 3 639 735
- FR-A1- 3 091 822
- US-B2- 9 669 172

## Description

L'invention concerne un dispositif de ventilation non-invasive (VNI), notamment à pression positive continue, à fonctionnement pneumatique pour traiter les patients souffrant de difficultés ou défaillances respiratoires, en particulier les personnes infectées par un coronavirus affectant ses capacités pulmonaires, par exemple le COVID-19 ou l'un de ses variants.

Des causes variées peuvent conduire à une défaillance du système respiratoire de certaines personnes, comme par exemple les infections virales, notamment celles engendrées par un coronavirus, tel le COVID-19.

Lorsque la défaillance du système respiratoire entraine une diminution de l'oxygène sanguin de la personne, c'est-à-dire une hypoxémie, et donc une diminution de l'oxygène dans ses tissus, la personne, i.e. le patient, risque un arrêt cardiaque hypoxique pouvant engendrer son décès.

Pour éviter cela, un traitement classique repose sur un apport d'oxygène supplémentaire au patient. L'oxygène peut lui être fourni simplement via un masque respiratoire alimenté par une source d'oxygène, telle une bouteille de gaz, ou en utilisant un ventilateur mécanique (i.e. un respirateur) fournissant l'oxygène à une interface respiratoire patient, tel qu'un masque respiratoire dans les cas simples ou une sonde d'intubation trachéale ou un dispositif supra-glottique dans les cas plus graves, voire critiques. Utiliser un ventilateur mécanique permet de fournir l'oxygène selon différents modes de ventilation.

Parfois, il est aussi possible de fournir l'oxygène au moyen de dispositifs délivrant le gaz à pression positive continue ou CPAP (pour *Continuous Positive Airway Pressure* en anglais). La pression positive continue, en plus de l'adjonction d'oxygène, permet de lutter contre l'atélectasie des petites voies aériennes, c'est-à-dire leur fermeture par « affaissement », mais aussi de diminuer le travail respiratoire du patient, c'est-à-dire l'effort que ce dernier doit fournir pour respirer, et ainsi améliore grandement la tolérance pour le patient.

Un dispositif de type CPAP donne donc de meilleurs résultats que les masques respiratoires simplement alimentés par une source d'oxygène et, par ailleurs, est généralement de conception et fonctionnement plus simples qu'un ventilateur mécanique, et son utilisation nécessite une formation moindre du personnel de santé.

A titre d'exemple, la pandémie liée au COVID-19 a mis en évidence un besoin important de pouvoir disposer de tels dispositifs de type CPAP pour traiter les patients infectés par ce virus ou l'un de ses variants.

Par ailleurs, on connait de FR-A-3091822 un ensemble de ventilation qui comprend une embase sur laquelle viennent se fixer un ventilateur médical équipé d'une micro-soufflante délivrant de l'air et un module de ventilation alimenté par le ventilateur médical. Le module de ventilation comprend un circuit de gaz à branches inspiratoire et expiratoire agencée en parallèle. La micro-soufflante du ventilateur médical aspire l'air ambiant avant de le fournir au circuit de gaz du module de ventilation. A cette fin, la sortie de gaz du ventilateur médical est directement connectée à l'entrée de gaz du circuit de gaz du module de ventilation. Or, un tel ensemble est de conception complexe et donc onéreux puisqu'il comprend une micro-soufflante, donc obligatoirement des moyens de pilotage de la micro-soufflante, des moyens d'alimentation électrique et autres composants indispensables à son fonctionnement. De plus, un tel ensemble est destiné à soigner les patients à domicile, c'est-à-dire en l'absence de tout personnel soignant. Il n'est donc pas conçu ou adapté aux soins hospitaliers, en particulier au traitement des patients infectés par un coronavirus, tel le COVID-19 ou l'un de ses variants.

La présente invention s'inscrit dans ce contexte et vise à proposer un dispositif de ventilation non-invasive (VNI) de patient de type CPAP qui soit de conception simple et de fonctionnement entièrement pneumatique de manière à pouvoir être fabriqué facilement, rapidement et/ou en grand nombre, et qui soit par ailleurs efficace pour traiter les patients souffrant de difficultés ou défaillances respiratoires, notamment ceux infectés par un coronavirus, tel le COVID-19, et aussi facile à utiliser de manière à pouvoir être mis en oeuvre par tout le personnel soignant, i.e. médecins, infirmiers, pompiers ou autres, sans nécessiter une formation spécifique poussée, en particulier en milieu hospitalier.

L'invention concerne alors un dispositif de ventilation non-invasive (VNI), notamment à pression continue positive (CPAP), comprenant :
- un module d'admission de gaz comprenant :
   ▪ un passage interne de gaz,
   ▪ un orifice d'injection d'oxygène et un orifice de fourniture de gaz en communication fluidique avec le passage interne de gaz,
   ▪ un réservoir de gaz ayant un volume interne d'au moins 15 L, fixé au module d'admission de gaz et relié fluidiquement au passage interne de gaz,
- un module de ventilation comprenant :
   ▪ un circuit de gaz interne comprenant un orifice d'entrée de gaz et un orifice de sortie de gaz,
   ▪ une valve d'échappement configurée pour permettre un échappement de gaz vers l'atmosphère via un orifice d'échappement pendant chaque phase expiratoire du patient et pour empêcher l'échappement de gaz vers l'atmosphère pendant chaque phase inspiratoire du patient, et
   ▪ un dispositif anti-retour de gaz agencé dans le circuit de gaz interne, et
- une ligne d'acheminement de gaz comprenant un conduit flexible ayant une longueur comprise entre 0,5 (i.e. 50 cm) et 10 mètres, reliant fluidiquement, via l'orifice de fourniture de gaz, le passage interne de gaz du module d'admission de gaz circuit de gaz interne du module de ventilation, via l'orifice d'entrée de gaz.

Selon le mode de réalisation considéré, le dispositif de ventilation non-invasive selon la technologie peut comprendre l'une ou plusieurs des caractéristiques suivantes :
- le module d'admission de gaz comprend un premier corps de module traversé par le passage interne de gaz.
- la ligne d'acheminement de gaz est reliée par une extrémité amont au module d'admission de gaz.
- la ligne d'acheminement de gaz est reliée par une extrémité aval au module de ventilation.
- la ligne d'acheminement de gaz comprend un conduit flexible, i.e. un tuyau souple, par exemple en polymère.
- la ligne d'acheminement de gaz comprend un conduit flexible ayant une longueur comprise entre 1 et 7 mètres, de préférence de moins de 5 mètres, de préférence encore de moins de 3 mètres, avantageusement entre 1 et 2 mètres.
- la ligne d'acheminement de gaz comprend des moyens de connexion agencés à ses extrémités amont et aval pour permettre un raccordement mécanique et fluidique de la ligne auxdits module d'admission de gaz et module de ventilation.
- la ligne d'acheminement de gaz achemine le gaz en direction du patient, c'est-à-dire dans le sens allant du module d'admission de gaz vers le module de ventilation.
- le réservoir de gaz a un volume interne d'au moins 18 Litres, avantageusement d'au moins 20 Litres (Litres donnés en équivalent en eau) .
- le réservoir de gaz a un volume interne inférieur ou égal à 50 L, de préférence inférieur ou égal à 40 L.
- avantageusement, le réservoir de gaz a un volume interne compris entre 25 et 35 L, typiquement de l'ordre de 30 L.
- le réservoir de gaz est raccordé mécaniquement et fluidiquement au module d'admission de gaz.
- la ligne d'acheminement de gaz est fixée mécaniquement par une extrémité amont au module d'admission de gaz et par une extrémité aval au module de ventilation, c'est-à-dire que les modules d'admission de gaz et ventilation sont séparées l'un de l'autre en étant raccordés mécaniquement et fluidiquement l'un à l'autre par ladite ligne d'acheminement de gaz, typiquement un tuyau flexible.
- la valve d'échappement comprend un compartiment interne, un élément d'obturation coopérant avec un siège de valve, un moyen élastique agencé dans le compartiment interne et agissant sur l'élément d'obturation de manière à le repousser vers le siège de valve et un orifice d'échappement relié fluidiquement à l'atmosphère.
- l'élément d'obturation est un clapet, une membrane flexible ou analogue.
- la valve d'échappement comprend un système de réglage de PEP configuré pour permettre à un utilisateur de régler un niveau de pression expiratoire positive (PEP) compris entre 0 et 30 cmH₂O, en modulant la force élastique que le moyen élastique exerce sur l'élément d'obturation pour le repousser contre le siège de valve.
- selon un autre mode de réalisation, la valve d'échappement comprend un système de PEP non réglable, c'est-à-dire configuré pour assurer une pression expiratoire positive (PEP) fixe, i.e. prédéfinie, comprise entre 0 et 30 cmH20, par exemple une PEP égale à 5 cmH20, 7,5 cmH20, 10 cmH20 ou toute autre valeur jusqu'à 30 cmH20.
- le module d'admission de gaz comprend en outre une entrée venturi configurée pour être raccordée fluidiquement à un dispositif à venturi, à savoir un système à venturi en communication avec l'atmosphère ambiante pour permettre une entrée d'air et/ou d'oxygène supplémentaire dans le passage interne de gaz, notamment en cas de pic de débit de gaz, en particulier un mélange gazeux air/O₂.
- l'entrée venturi et/ou le réservoir de gaz sont reliés fluidiquement au passage interne de gaz du corps de module du module d'admission de gaz.
- un dispositif à venturi est connecté à l'entrée venturi du module d'admission de gaz.
- alternativement et préférentiellement, l'entrée venturi est configurée pour former en outre l'orifice d'injection d'oxygène de manière à permettre un raccordement fluidique (et mécanique) soit d'une source d'oxygène, soit d'un dispositif à venturi. Autrement dit, l'orifice d'injection d'oxygène et l'entrée venturi sont communes et uniques pour permettre d'y fixer, selon le cas, une source d'oxygène ou un dispositif à venturi.
- le dispositif à venturi est monté à demeure ou de manière détachable.
- le dispositif à venturi comprend un corps de venturi comprenant au moins une entrée d'air venturi (i.e. une ou plusieurs orifices ou analogues) permettant l'entrée d'air atmosphérique dans le dispositif à venturi et au moins une entrée d'oxygène venturi permettant l'entrée d'oxygène dans le dispositif à venturi de manière à pouvoir réaliser un (des) mélange air/O₂ dans le corps de venturi.
- le corps de venturi comprenant au moins une sortie venturi pour fournir (i.e. alimenter) le mélange air/O₂ au passage interne de gaz du module d'admission de gaz, via une entrée venturi agencée sur le module d'admission de gaz.
- une source d'oxygène est raccordée fluidiquement à l'orifice d'injection d'oxygène du module d'admission de gaz.
- la ligne d'acheminement de gaz comprend un tuyau flexible, i.e. souple, typiquement polymère.
- le dispositif anti-retour de gaz comprend une valve unidirectionnelle, c'est-à-dire une valve autorisant seulement la circulation du gaz dans le sens allant vers le patient, donc s'opposant à toute remontée de gaz vers la ligne d'acheminement de gaz.
- le réservoir de gaz est relié fluidiquement au passage interne de gaz via un orifice de liaison du module d'admission de gaz.
- le premier corps de module du module d'admission de gaz comprenant des premiers moyens de raccordement configurés pour permettre d'y raccorder une source d'oxygène, le réservoir de gaz et la ligne d'acheminement de gaz.
- le module de ventilation comprend un second corps de module comprenant des seconds moyens de raccordement configurés pour permettre d'y raccorder la ligne d'acheminement de gaz.
- le premier et/ou le second corps de module sont formés, au moins en partie, en matériau rigide, de préférence en polymère, en particulier formés chacun d'une pièce unique, en particulier obtenue par moulage par injection ou toute autre technique adéquate.
- les premiers et/ou les seconds moyens de raccordement comprennent des connecteurs, des raccords ou analogues, notamment à raccordement par emboitement, vissage, baïonnette ou tout autre système de fixation.
- les moyens de connexion agencés aux extrémités amont et aval de la ligne d'acheminement de gaz sont configurés pour se coupler aux premiers et/ou les seconds moyens de raccordement servant à raccorder la ligne d'acheminement de gaz.
- le siège de valve de la valve d'échappement coopère avec l'élément d'obturation pour assurer une étanchéité fluidique entre eux.
- le moyen élastique repousse normalement l'élément d'obturation contre le siège de valve.
- l'élément d'obturation est en métal, en polymère ou autre.
- l'élément d'obturation est une membrane flexible ayant une forme de disque « à soufflets » ou autre.
- le moyen élastique comprend un ressort ou analogue.
- l'élément d'obturation est fixé à la paroi interne du corps de valve.
- une interface respiratoire est reliée fluidiquement au module de ventilation, c'est-à-dire en communication fluidique avec le circuit de gaz interne.
- le module de ventilation comprend des moyens de raccordement d'interface agencés au niveau de l'orifice de sortie de gaz.
- l'interface respiratoire est raccordée fluidiquement aux moyens de raccordement d'interface du module de ventilation, par exemple un connecteur, raccord ou analogue, notamment à raccordement par emboitement, vissage, baïonnette ou tout autre système de fixation.
- l'interface respiratoire comprend un masque respiratoire, par exemple nasal ou facial (i.e. bucco-nasal), une sonde d'intubation trachéale ou un dispositif supra-glottique.
- le corps de valve a une forme générale cylindrique ou analogue.
- le système de réglage est porté par le corps de valve de la valve d'échappement.
- le système de réglage comprend une partie mobile en forme de capuchon venant se fixer par vissage au corps de valve, c'est-à-dire que le capuchon surmonte le corps de valve.
- le moyen élastique, i.e. ressort ou analogue, vient prendre appui dans le fond de la partie mobile en forme de capuchon.
- le réservoir de gaz comprend une enveloppe souple, de préférence formée de polymère.
- le réservoir de gaz est flexible, i.e. déformable.
- le réservoir de gaz comprend une enveloppe souple en polymère biocompatible sans phtalate.
- le module d'admission de gaz comprend en outre une valve de sécurité configurée pour autoriser une entrée d'air dans le passage interne de gaz du module d'admission en cas d'insuffisance de gaz dans le réservoir et/ou provenant de la source d'oxygène et/ou du dispositif à venturi.
- une source d'oxygène est reliée fluidiquement aux premiers moyens de raccordement agencés sur le premier corps de module du module d'admission de gaz.
- une (autre) source d'oxygène est reliée fluidiquement au dispositif à venturi.
- la (ou les) source d'oxygène comprend une bouteille d'oxygène sous pression ou une canalisation d'amenée d'oxygène débouchant au niveau d'une prise de raccordement, typiquement une prise murale.

De plus, le dispositif de ventilation non-invasive (VNI) est configuré, i.e. conçu, pour fonctionner de manière, entièrement et uniquement, pneumatique, c'est-à-dire qu'il ne comprend aucune micro-soufflante (i.e. turbine ou compresseur) interne ou analogue, et sans courant électrique, c'est-à-dire qu'il ne comprend aucune source de courant électrique.

L'invention va maintenant être mieux comprise grâce à la description détaillée suivante, faite à titre illustratif mais non limitatif, en référence aux figures annexées parmi lesquelles :
Fig. 1 schématise un mode de réalisation d'un dispositif de VNI selon l'invention ;
Fig. 2 schématise un mode de réalisation de la valve d'échappement pneumatique d'un dispositif de VNI selon l'invention, en particulier le dispositif de Fig. 1 ; et
Fig. 3 schématise les performances ventilatoires en fonction du volume du réservoir de gaz faisant office de réserve d'oxygène disponible pour le patient.

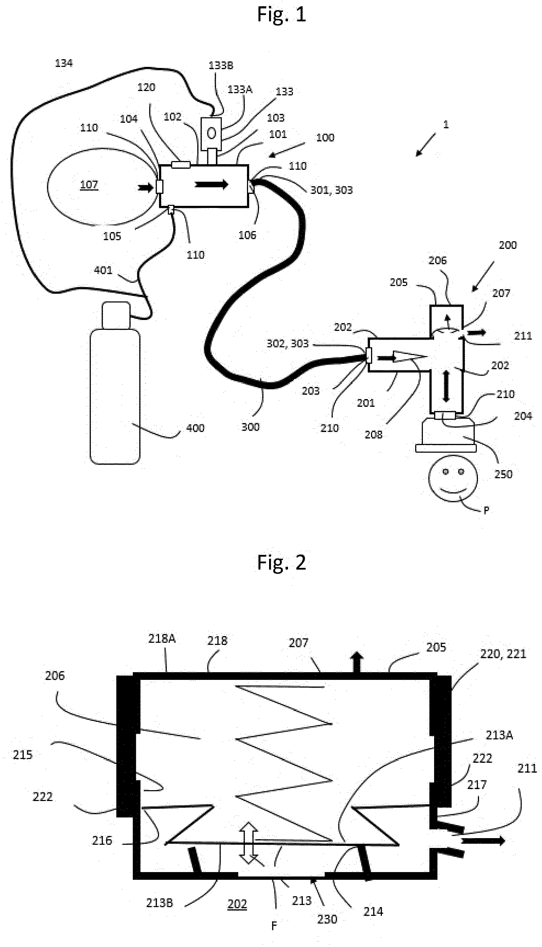

Fig. 1 schématise un mode de réalisation d'un dispositif 1 de ventilation non-invasive ou VNI à fonctionnement pneumatique selon l'invention délivrant du gaz respiratoire, de préférence à pression continue positive (CPAP), aux voies respiratoires d'un patient P souffrant de difficultés ou défaillances respiratoires, par exemple une personne infectée par un coronavirus, tel le COVID-19, ou résultant de toute autre cause ou pathologie, en particulier pour une utilisation en milieu hospitalier.

Selon l'invention, le dispositif 1 de VNI comprend un module d'admission de gaz 100, un module de ventilation 200 et une ligne d'acheminement de gaz 300, à savoir un tuyau flexible, reliant fluidiquement le passage interne de gaz 102 du module d'admission de gaz 100 au circuit de gaz interne 202 du module de ventilation 200 de manière à pouvoir acheminer du gaz respiratoire sous pression, typiquement de l'oxygène ou un mélange air/oxygène depuis le module d'admission de gaz 100 jusqu'au module de ventilation 200, lequel permet de fournir le gaz au patient P, via une interface respiratoire patient 250, tel un masque respiratoire, e.g. facial, ou une sonde d'intubation trachéale ou un dispositif supra-glottique par exemple.

Plus précisément, le module d'admission de gaz 100 comprend un premier corps de module 101 en matériau rigide, de type polymère par exemple, traversé par un passage interne de gaz 102.

Le module d'admission de gaz 100, typiquement le premier corps de module 101, comprend un orifice d'injection d'oxygène 105 alimenté en oxygène gazeux par une source d'oxygène 400 et un orifice de fourniture de gaz 106 pour évacuer le gaz sous pression se trouvant dans le passage interne de gaz 102 vers la ligne d'acheminement de gaz 300 qui vient se raccorder au module d'admission de gaz 100 par une extrémité amont 301.

La ligne d'acheminement de gaz 300 permet de véhiculer le gaz au sein de son lumen depuis le module d'admission de gaz 100 jusqu'au module de ventilation 200, c'est-à-dire en direction du patient P, auquel elle est reliée par une extrémité aval 302. Préférentiellement, la ligne d'acheminement de gaz 300 est une conduite de gaz flexible, c'est-à-dire un tuyau souple, par exemple en polymère. Elle comprend des moyens de connexion 303, agencés à ses extrémités amont 301 et aval 302, tels des connecteurs de raccordement mécanique et fluidique par exemple. De préférence, elle a une longueur comprise entre 50 cm et 5 mètres, typiquement entre 1 et 4 m, en général de moins de 2 m.

La source d'oxygène 400 comprend une bouteille d'oxygène sous pression, comme illustré en Fig. 1, ou, selon un autre mode de réalisation (non montré), une canalisation d'amenée d'oxygène débouchant au niveau d'une prise de raccordement, typiquement une prise murale, laquelle peut faire partie d'un réseau de canalisations de gaz agencé dans un bâtiment hospitalier ou analogue.

Par ailleurs, le passage interne de gaz 102 du module d'admission de gaz 100 est aussi en communication fluidique avec un réservoir de gaz 107 pour l'alimenter en gaz, typiquement en oxygène, via un orifice de liaison 104 agencé dans la paroi du module d'admission de gaz 100, i.e. dans le premier corps de module.

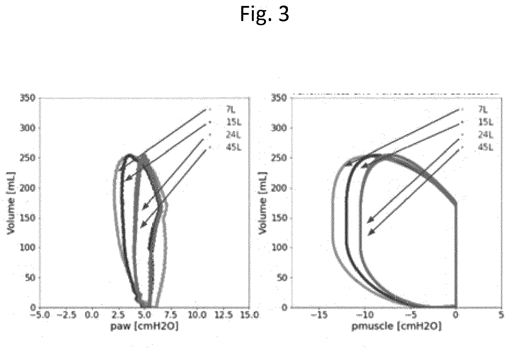

Le réservoir de gaz 107 forme une réserve d'oxygène gazeux, c'est-à-dire une capacité tampon, permettant de répondre à la demande inspiratoire du patient P, c'est-à-dire d'assurer une bonne oxygénation du patient, même si celui-ci est en forte demande. Le réservoir de gaz 107 est par exemple formé d'une enveloppe souple en polymère ayant une contenance d'au moins 15 Litres, avantageusement d'au moins 20 Litres, typiquement de l'ordre de 23 à 35 Litres, par exemple de 25 à 30 Litres environ (contenance en L en équivalent eau). L'importance de la taille du réservoir de gaz 107 faisant office de réserve d'oxygène à disposition du patient est montrée sur la Fig. 3 et expliquée ci-dessous.

Préférentiellement, le module d'admission de gaz 100 peut comprendre en outre une entrée venturi 103 pouvant être reliée fluidiquement à un dispositif à venturi 133 comprenant (au moins) une entrée d'air venturi 133A pour de l'air atmosphérique et une entrée d'oxygène venturi 133B venant se raccorder, via une ligne d'oxygène 134, à une source d'oxygène permettant de réaliser un mélange air/oxygène qui alimente ensuite le passage interne de gaz 102 du module d'admission de gaz 100.

Dans le mode de réalisation de Fig. 1, l'orifice d'injection d'oxygène 105 et l'entrée venturi 103 sont deux orifices ou entrées distinctes l'une de l'autre.

Cependant, selon un autre mode de réalisation (non montré), l'orifice d'injection d'oxygène 105 et l'entrée venturi 103 peuvent être formés d'un unique orifice ou entrée. Autrement dit, l'entrée venturi 103 est configurée pour former aussi l'orifice d'injection d'oxygène 105 de manière à y connecter fluidiquement et mécaniquement soit une source d'oxygène, soit un dispositif à venturi 133.

Dans le mode de réalisation proposé, la source d'oxygène est la même source d'oxygène 400, par exemple une bouteille d'oxygène sous pression, que celle alimentant en oxygène, le passage interne de gaz 102 du module d'admission de gaz 100 via l'orifice d'injection d'oxygène 105 ; toutefois, il pourrait s'agir d'une autre source d'oxygène comme une seconde bouteille de gaz, un concentrateur d'oxygène ou encore une canalisation de gaz.

Le dispositif à venturi 133 peut être monté de manière détachable. Il est n'est pas indispensable mais peut s'avérer très utile lorsque l'on souhaite économiser de l'oxygène puisqu'il permet de réaliser des mélanges O2/air, donc de diluer l'oxygène avec de l'air ambiant, donc d'en utiliser une quantité moindre.

Autrement dit, le module d'admission de gaz 100 peut comprendre aussi un dispositif ou système à venturi 133, agencé à demeure ou venant se fixer sur le premier corps de module 101, qui est en communication avec l'atmosphère ambiante via son (ou ses) entrée d'air venturi 133A pour permettre une entrée d'air supplémentaire dans le passage interne de gaz 102, notamment en cas de pic de débit de gaz ou lorsqu'on souhaite obtenir un mélange air/O₂ plutôt qu'utiliser de l'O₂ pur, de sorte de pouvoir augmenter le débit inspiratoire au détriment d'une concentration en oxygène élevée.

L'orifice d'injection d'oxygène 105, l'orifice de fourniture de gaz 106 et l'orifice de liaison 104 peuvent être portés par des premiers moyens de raccordement 110, par exemple des connecteurs, des raccords ou analogues, permettant d'y raccorder la source d'oxygène 400 via un tuyau souple 401, le réservoir de gaz 107 et la ligne d'acheminement de gaz 300 via son connecteur amont 301, 303. Leur raccordement peut se faire par emboitement, vissage, baïonnette ou autre. Il en va de même de l'entrée venturi 103 à laquelle peut venir se raccorder un dispositif à venturi 133.

Le module d'admission de gaz 100 comprend en outre une valve de sécurité 120 conçue pour autoriser une entrée d'air ambiant dans le passage interne de gaz 102 du module d'admission 100 en cas d'insuffisance de gaz dans le réservoir 107 et/ou provenant de la source d'oxygène 400, c'est-à-dire lorsque la quantité de gaz disponible pour le patient P est insuffisante, par exemple en cas de défaillance ou dysfonction de l'un ou l'autre de ces composants. La valve de sécurité 120 permet donc d'éviter une asphyxie du patient P en lui fournissant de l'air ambiant de secours.

Par ailleurs, le module de ventilation 200 comprend un deuxième corps de module 201 traversé par un circuit de gaz interne 202, c'est-à-dire un conduit, passage ou autre, comprenant un orifice d'entrée de gaz 203 alimenté en gaz par la ligne d'acheminement de gaz 300, telle une conduite ou tuyau de gaz flexible souple, et un orifice de sortie de gaz 204 pour fournir le gaz sous pression au patient P, via l'interface respiratoire 250.

Là encore, le deuxième corps de module 201 du module de ventilation 200 peut être formé d'un matériau rigide de type polymère par exemple.

Comme précédemment, les orifice d'entrée de gaz 203 et orifice de sortie de gaz 204 peuvent être portés là aussi par des seconds moyens de raccordement 210, par exemple des connecteurs, des raccords ou analogues, de type à emboitement, vissage, baïonnette ou autre, auxquels vient se raccorder mécaniquement et fluidiquement la ligne d'acheminement de gaz 300 via son extrémité aval 302 munie d'un connecteur aval 303, et soit directement, soit indirectement l'interface respiratoire 250.

Un dispositif anti-retour de gaz 208 agencé dans le circuit de gaz interne 202, approximative de l'orifice d'entrée de gaz 203. Préférentiellement, le dispositif anti-retour de gaz 208 comprend une valve unidirectionnelle autorisant le passage de gaz uniquement dans un sens, à savoir en direction du patient P, et donc s'opposant aux remontées de gaz, tels les gaz expirés par le patient P, vers et dans la ligne d'acheminement de gaz 300 et le module d'admission de gaz 100.

Afin de pouvoir évacuer à l'atmosphère, les gaz expirés par le patient P, est prévue une valve d'échappement 205 agencée dans le module de ventilation 200. Elle est conçue pour permettre l'échappement des gaz expirés vers l'atmosphère via un orifice d'échappement 207 pendant les phases expiratoires du patient P et, à l'inverse, pour empêcher l'échappement de gaz vers l'atmosphère pendant les phases inspiratoires du patient P.

Un mode de réalisation (vue en coupe) d'une valve d'échappement 205 est schématisé en Fig. 2.

Comme on le voit, la valve d'échappement pneumatique 205 comprend un compartiment interne 206 comprenant un élément d'obturation 213, tel un clapet ou une membrane flexible, un moyen élastique 207, tel un ressort, agencé dans le compartiment interne 206 et agissant sur l'élément d'obturation 213 de manière à appuyer sur sa face interne 213A et le repousser normalement vers un siège de valve 214, pendant les phases inspiratoires. Le siège de valve 214 coopère avec la face externe 213B de l'élément d'obturation 213 pour assurer une étanchéité fluidique entre eux, pendant chaque phase inspiratoire du patient.

Dans le mode de réalisation illustré en Fig. 2, l'élément d'obturation est par exemple une membrane flexible 213 en métal, plastique ou un autre matériau. Elle a par exemple une forme générale de « disque à soufflets » et est fixée (en 216) à la paroi interne 215 du corps de valve 217 de la valve d'échappement 205. Toutefois, selon d'autres modes de réalisation, l'élément d'obturation peut être un clapet, par exemple rigide, ou tout moyen ou système d'obturation équivalent.

L'élément d'obturation 213 a sa face interne 213A du côté du compartiment interne 206 et sa face externe 213B orientée vers le circuit de gaz interne 202. Autrement dit, l'élément d'obturation 213 sépare le compartiment interne 206 du circuit de gaz interne 202 en obturant normalement l'entrée de valve 230.

Dans le mode de réalisation présenté, le siège de valve 214 est agencé autour de l'entrée de valve 230. Le siège de valve 214 a par exemple une forme annulaire et l'élément d'obturation 213 faisant office de clapet de valve peut avoir une forme de disque.

L'élément d'obturation se déplace et/ou se déforme en rapprochement ou en éloignement (sens de la flèche F) en fonction des phases inspiratoires et expiratoires du patient et sous l'effet de la force élastique exercée par le moyen élastique 207, i.e. ressort, comme expliqué ci-après,

Afin de permettre le fonctionnement de la valve d'échappement 205, le compartiment interne 206 comprend aussi un orifice d'échappement de gaz 211 servant à évacuer le gaz vers l'atmosphère ambiante lorsque la valve d'échappement 205 s'ouvre, c'est-à-dire lorsque l'élément d'obturation se déplace ou se déforme en éloignement du siège de valve 214, durant les phases expiratoires du patient, étant donné que la pression du gaz expiré par le patient va aller s'exercer sur la face interne 213A et le repousser vers le haut sur la Fig. 2, c'est-à-dire vers l'intérieur du compartiment interne 206 de la valve 205.

Autrement dit, la pression du gaz expiré par le patient doit être supérieure à la force de répulsion, c'est-à-dire la raideur, du moyen élastique 207, i.e. ressort, pour pouvoir obtenir une ouverture de la valve d'échappement 205, c'est-à-dire un décollement de l'élément d'obturation 213 du siège de valve 214, une libération de l'entrée de valve 230 et donc une mise en communication fluidique du circuit de gaz interne 202 avec l'atmosphère ambiante via successivement l'entrée de valve 230 et l'orifice d'échappement de gaz 211.

En effet, en se décollant du siège de valve 214 sous l'effet de la pression du gaz expiré, l'élément d'obturation 213 libère un passage entre sa face interne 213A et le siège de valve 214, pour le gaz sous pression contenu dans le circuit de gaz interne 202, lequel peut alors circuler au travers de l'entrée de valve 230 et ensuite être évacué vers l'atmosphère via l'orifice d'échappement de gaz 211. Il est primordial que, lorsque le patient P expire, la (quasi)totalité du gaz expiré riche en CO₂ soit évacuée vers l'atmosphère afin d'éviter que, lors de l'inspiration suivante, le patient ne le ré-inhale.

Cette évacuation du gaz expiré riche en CO₂ se fait au travers de la valve d'expiration 205 qui s'ouvre, pendant les phases expiratoires, en autorisant le passage de gaz au travers de l'orifice d'échappement de gaz 211.

Par ailleurs, le dispositif anti-retour 208 participe à une évacuation efficace du gaz hors du module de ventilation 200, pendant les phases d'expiration du patient, en empêchant les remontées de gaz riche en CO₂ dans la ligne d'acheminement de gaz 300.

Dans le mode de réalisation de Fig. 2, la valve d'échappement 205 comprend un système de réglage de PEP 218 permettant à l'utilisateur d'ajuster ou de fixer la force élastique que le moyen élastique 207, i.e. ressort ou analogue, va appliquer sur la surface ou face interne 213A de l'élément d'obturation 213 pour le repousser contre le siège de valve 214. Le système de réglage 218 permet donc de fixer un niveau de pression expiratoire positive (PEP) compris entre 0 et 30 cmH₂O.

Le système de réglage de PEP 218 est porté par le corps de valve 217 de la valve d'échappement 205 qui a une forme générale cylindrique ou analogue. Le système de réglage 218 comprend une partie mobile 220 en forme de capuchon venant se fixer par vissage 221, par exemple via un système de type filetage/taraudage, à baïonnette ou analogue, au corps de valve 217. Le moyen élastique 207, i.e. ressort ou analogue, vient prendre appui dans le fond 218A de la partie mobile 220 en forme de capuchon. Autrement dit, le moyen élastique 207 est pris en sandwich entre le fond 218A de la partie mobile 220 et l'élément d'obturation 213 coopérant avec le siège de valve 214.

Ainsi, un vissage de la partie mobile 220 sur le corps de valve 217 va engendrer un rapprochement de cette partie mobile 220 de l'élément d'obturation 213 et par conséquent un écrasement ou compression du moyen élastique 207, typiquement une augmentation de sa raideur, donc aussi une augmentation du niveau de PEP.

A l'inverse, un dévissage de la partie mobile 220 va engendrer un éloignement de la partie mobile 220 de l'élément d'obturation 213 et par conséquent un relâchement ou décompression du moyen élastique 207, typiquement une diminution de sa raideur, donc une baisse du niveau de PEP.

Toutefois, selon un autre mode de réalisation (non montré), la valve d'échappement 205 peut comprend un système de PEP non réglable, c'est-à-dire permettant d'assurer une pression expiratoire positive (PEP) prédéfinie entre 0 et 30 cmH20, par exemple une PEP égale à 5 cmH20, 7,5 cmH20, 10 cmH20 ou autre.

Les courbes de Fig. 3 permettent de montrer l'efficacité d'une ventilation en fonction de la taille du réservoir de gaz 107 utilisé. Ces courbes ont été obtenues lors de tests opérés sur un banc de tests de type ASL5000, lequel est capable de simuler la respiration spontanée d'un individu (sain ou malade) en fonction de paramètres prédéfinis.

En abscisses, sont données la "paw" représentant la pression à la bouche du patient (graphique de gauche sur Fig. 3) et la "pmuscle" représentant la pression musculaire que doit fournir le patient (graphique de droite sur Fig. 3) et, en ordonnées, le volume généré (en mL) en fonction des pressions.

Comme on le voit, la taille du réservoir d'oxygène (en L) impacte directement les performances ventilatoires du système puisqu'on observe que plus le réservoir est petit, plus les variations de pression (i.e. *paw* et *pmuscle*) sont élevées pour atteindre le volume réglé. Cela signifie qu'un patient doit fournir un plus grand effort respiratoire afin de respirer convenablement. En d'autres termes, à partir de 15 à 20 L de contenance du réservoir, il y a un réel bénéfice clinique pour le patient.

Autrement dit, la taille du réservoir de gaz 107 est importante puisque l'efficacité de la ventilation croit avec la capacité du réservoir de gaz 107. Si la ventilation est possible avec une capacité de quelques litres, on constate que les résultats sont meilleurs pour une capacité d'au moins 10 à 12 L, avantageusement supérieure à 15 L. Toutefois, augmenter la capacité du réservoir de gaz 107 au-delà de 40 à 45 L ne permet pas de noter une améliorer supplémentaire de la ventilation. De là, on utilise préférentiellement un réservoir de gaz 107 ayant une capacité comprise entre 15 et 35 L environ, avantageusement de l'ordre de 25 à 30 L.

D'une façon générale, le dispositif 1 de VNI de l'invention permet d'assurer le maintien d'une pression continue positive (CPAP) pendant la ventilation du patient, donc d'une bonne FiO₂, à savoir la Fraction Inspirée en Oxygène, qui est la concentration d'oxygène que le patient va recevoir in fine. Ainsi, il est important de pouvoir fournir au patient, une FiO₂ proche de 100% (c'est-à-dire proche de l'oxygène pur) pour obtenir une bonne oxygénation de son sang, grâce à la ventilation, en particulier chez les patients infectés par le Covid-19 ou tout autre virus ou analogue affectant négativement ses capacités pulmonaires.

## Revendications

1. Dispositif (1) de ventilation non-invasive (VNI), notamment à pression continue positive (CPAP), comprenant :
- un module d'admission de gaz (100) comprenant :
▪ un passage interne de gaz (102),
▪ un orifice d'injection d'oxygène (105) et un orifice de fourniture de gaz (106) en communication fluidique avec le passage interne de gaz (102),
▪ un réservoir de gaz (107) ayant un volume interne d'au moins 15 L, fixé au module d'admission de gaz (100) et relié fluidiquement au passage interne de gaz (102),
- un module de ventilation (200) comprenant :
▪ un circuit de gaz interne (202) comprenant un orifice d'entrée de gaz (203) et un orifice de sortie de gaz (204),
▪ une valve d'échappement (205) configurée pour permettre un échappement de gaz vers l'atmosphère via un orifice d'échappement (207) pendant chaque phase expiratoire du patient et pour empêcher l'échappement de gaz vers l'atmosphère pendant chaque phase inspiratoire du patient, et
▪ un dispositif anti-retour de gaz (208) agencé dans le circuit de gaz interne (202), et
- une ligne d'acheminement de gaz (300) comprenant un conduit flexible ayant une longueur comprise entre 0.5 et 10 mètres, reliant fluidiquement, via l'orifice de fourniture de gaz (106), le passage interne de gaz (102) du module d'admission de gaz (100) au circuit de gaz interne (202) du module de ventilation (200), via l'orifice d'entrée de gaz (204).

2. Dispositif selon la revendication 1, **caractérisé en ce que** le réservoir de gaz (107) a un volume interne d'au moins 20 L.

3. Dispositif selon l'une des revendications précédentes, **caractérisé en ce que** le réservoir de gaz (107) a un volume interne inférieur ou égal à 50 L, de préférence inférieur ou égal à 40 L.

4. Dispositif selon la revendication 1, **caractérisé en ce que** le module d'admission de gaz (100) comprend une entrée venturi (103) configurée pour être raccordée fluidiquement à un dispositif à venturi (133).

5. Dispositif selon l'une des revendications précédentes, **caractérisé en ce que** le module d'admission de gaz (100) comprend un corps de module (101) traversé par le passage interne de gaz (102), l'entrée venturi (103) et/ou le réservoir de gaz (107) étant reliés fluidiquement au passage interne de gaz (102) du corps de module (101) du module d'admission de gaz (100).

6. Dispositif selon la revendication 1, **caractérisé en ce que** le dispositif anti-retour de gaz (208) du module de ventilation (200) comprend une valve unidirectionnelle.

7. Dispositif selon la revendication 1, **caractérisé en ce que** la valve d'échappement (205) comprend un compartiment interne (206), un élément d'obturation (213) coopérant avec un siège de valve (214), un moyen élastique (207) agencé dans le compartiment interne (206) et agissant sur l'élément d'obturation (213) de manière à le repousser vers le siège de valve (214) et un orifice d'échappement (211) relié fluidiquement à l'atmosphère.

8. Dispositif selon la revendication 6, **caractérisé en ce que** la valve d'échappement (205) comprend :
- un système de réglage (218) de PEP configuré pour permettre à un utilisateur de régler un niveau de pression expiratoire positive (PEP) compris entre 0 et 30 cmH₂O, en modulant la force élastique que le moyen élastique (207) exerce sur l'élément d'obturation (213) pour le repousser contre le siège de valve (214), ou
- un système de PEP non réglable configuré pour assurer une pression expiratoire positive (PEP) fixe comprise entre 0 et 30 cmH20.

9. Dispositif selon la revendication 1, **caractérisé en ce que** le module de ventilation (200) comprend des moyens de raccordement (210) à une interface respiratoire (250) agencés au niveau de l'orifice de sortie de gaz (204).

10. Dispositif selon la revendication 3, **caractérisé en ce qu'**un dispositif à venturi (133) est connecté à l'entrée venturi (103) du module d'admission de gaz (100).

11. Dispositif selon l'une des revendications précédentes, **caractérisé en ce que** :
- une interface respiratoire (250) est raccordée fluidiquement aux moyens de raccordement (210) du module de ventilation (200),
- une source d'oxygène (400) est raccordée fluidiquement à l'orifice d'injection d'oxygène (105) du module d'admission de gaz (100) et/ou
- la ligne d'acheminement de gaz (300) comprend un tuyau flexible.

12. Dispositif selon la revendication 4, **caractérisé en ce que** l'entrée venturi (103) est configurée pour former en outre l'orifice d'injection d'oxygène (105) de manière à permettre un raccordement fluidique soit d'une source d'oxygène, soit d'un dispositif à venturi (133).

13. Dispositif selon la revendication 1, **caractérisé en ce que** le conduit flexible a une longueur comprise entre 1 et 5 mètres.

14. Dispositif selon la revendication 1, **caractérisé en ce que** la ligne d'acheminement de gaz (300) est fixée mécaniquement par une extrémité amont au module d'admission de gaz (100) et une extrémité aval au module de ventilation (200).

15. Dispositif selon l'une des revendications 1 à 3, **caractérisé en ce que** le réservoir de gaz (107) est en polymère et déformable.

## Patentansprüche

1. Vorrichtung (1) zur nichtinvasiven Beatmung (NIV), insbesondere mit kontinuierlichem positivem Atemwegsdruck (CPAP), umfassend:
- ein Gaseinlassmodul (100), umfassend:
• einen internen Gasdurchlass (102),
• eine Sauerstoffinjektionsöffnung (105) und eine Gaszufuhröffnung (106), die mit dem internen Gasdurchlass (102) in Fluidverbindung steht,
• einen Gasbehälter (107) mit einem Innenvolumen von mindestens 15 1, der am Gaseinlassmodul (100) befestigt und fluidisch mit dem internen Gasdurchlass (102) verbunden ist,
- ein Beatmungsmodul (200), umfassend:
• einen internen Gaskreislauf (202) mit einer Gaseinlassöffnung (203) und einer Gasauslassöffnung (204),
• ein Auslassventil (205), das dazu konfiguriert ist, während jeder exspiratorischen Phase des Patienten ein Entweichen von Gas über eine Auslassöffnung (207) in die Atmosphäre zu ermöglichen und während jeder inspiratorischen Phase des Patienten das Entweichen von Gas in die Atmosphäre zu verhindern, und
• eine Gasrückstromsperre (208), die im internen Gaskreislauf (202) angeordnet ist, und
- eine Gasförderleitung (300), die eine flexible Leitung mit einer Länge zwischen 0,5 und 10 Metern umfasst, die den internen Gasdurchlass (102) des Gaseinlassmoduls (100) über die Gaszufuhröffnung (106) fluidisch mit dem internen Gaskreislauf (202) des Beatmungsmoduls (200) über die Gaseinlassöffnung (204) verbindet.

2. Vorrichtung nach Anspruch 1, **dadurch gekennzeichnet, dass** der Gasbehälter (107) ein Innenvolumen von mindestens 20 1 aufweist.

3. Vorrichtung nach einem der vorangehenden Ansprüche, **dadurch gekennzeichnet, dass** der Gasbehälter (107) ein Innenvolumen von 50 1 oder weniger, vorzugsweise 40 1 oder weniger, aufweist.

4. Vorrichtung nach Anspruch 1, **dadurch gekennzeichnet, dass** das Gaseinlassmodul (100) einen Venturi-Einlass (103) umfasst, der dazu konfiguriert ist, fluidisch mit einer Venturi-Vorrichtung (133) verbunden zu sein.

5. Vorrichtung nach einem der vorangehenden Ansprüche, **dadurch gekennzeichnet, dass** das Gaseinlassmodul (100) einen Modulkörper (101) umfasst, durch den der interne Gasdurchlass (102) verläuft, wobei der Venturi-Einlass (103) und/oder der Gasbehälter (107) fluidisch mit dem internen Gasdurchlass (102) des Modulkörpers (101) des Gaseinlassmoduls (100) verbunden sind.

6. Vorrichtung nach Anspruch 1, **dadurch gekennzeichnet, dass** die Gasrückstromsperre (208) des Beatmungsmoduls (200) ein Einwegventil umfasst.

7. Vorrichtung nach Anspruch 1, **dadurch gekennzeichnet, dass** das Auslassventil (205) eine interne Kammer (206), ein Verschlusselement (213), das mit einem Ventilsitz (214) zusammenwirkt, ein elastisches Mittel (207), das in der internen Kammer (206) angeordnet ist und auf das Verschlusselement (213) einwirkt, um es in Richtung des Ventilsitzes (214) zu drücken, und eine fluidisch mit der Atmosphäre verbundene Auslassöffnung (211) umfasst.

8. Vorrichtung nach Anspruch 6, **dadurch gekennzeichnet, dass** das Auslassventil (205) Folgendes umfasst:
- ein PEEP-Einstellsystem (218), das dazu konfiguriert ist, es einem Benutzer zu ermöglichen, einen Pegel des positiven endexspiratorischen Drucks (PEEP) zwischen 0 und 30 cmH₂O einzustellen, indem die elastische Kraft, die das elastische Mittel (207) auf das Verschlusselement (213) ausübt, um es gegen den Ventilsitz (214) zu drücken, moduliert wird, oder
- ein nicht einstellbares PEEP-System, das dazu konfiguriert ist, einen festen positiven endexspiratorischen Druck (PEEP) zwischen 0 und 30 cmH₂0 zu gewährleisten.

9. Vorrichtung nach Anspruch 1, **dadurch gekennzeichnet, dass** das Beatmungsmodul (200) Mittel (210) zur Verbindung mit einer Atemschnittstelle (250) umfasst, die an der Gasauslassöffnung (204) angeordnet sind.

10. Vorrichtung nach Anspruch 3, **dadurch gekennzeichnet, dass** eine Venturi-Vorrichtung (133) mit dem Venturi-Einlass (103) des Gaseinlassmoduls (100) verbunden ist.

11. Vorrichtung nach einem der vorangehenden Ansprüche, **dadurch gekennzeichnet, dass**:
- eine Atemschnittstelle (250) fluidisch mit den Verbindungsmitteln (210) des Beatmungsmoduls (200) verbunden ist,
- eine Sauerstoffquelle (400) fluidisch mit der Sauerstoffinjektionsöffnung (105) des Gaseinlassmoduls (100) verbunden ist und/oder
- die Gasförderleitung (300) einen flexiblen Schlauch umfasst.

12. Vorrichtung nach Anspruch 4, **dadurch gekennzeichnet, dass** der Venturi-Einlass (103) dazu konfiguriert ist, ferner eine Sauerstoffinjektionsöffnung (105) zu bilden, um eine Fluidverbindung entweder mit einer Sauerstoffquelle oder mit einer Venturi-Vorrichtung (133) zu ermöglichen.

13. Vorrichtung nach Anspruch 1, **dadurch gekennzeichnet, dass** die flexible Leitung eine Länge zwischen 1 und 5 Metern aufweist.

14. Vorrichtung nach Anspruch 1, **dadurch gekennzeichnet, dass** die Gasförderleitung (300) mit einem stromaufwärtigen Ende mechanisch am Gaseinlassmodul (100) und mit einem stromabwärtigen Ende mechanisch am Beatmungsmodul (200) befestigt ist.

15. Vorrichtung nach einem der Ansprüche 1 bis 3, **dadurch gekennzeichnet, dass** der Gasbehälter (107) aus Polymer gefertigt und verformbar ist.

## Claims

1. Device (1) for non-invasive ventilation (NIV), in particular at a continuous positive airway pressure (CPAP), comprising:
- a gas admission module (100) comprising:
▪ an internal gas passage (102),
▪ an oxygen injection port (105) and a gas supply port (106) in fluidic communication with the internal gas passage (102),
▪ a gas reservoir (107) having an internal volume of at least 15 1, fixed to the gas admission module (100) and fluidically connected to the internal gas passage (102),
- a ventilation module (200) comprising:
▪ an internal gas circuit (202) comprising a gas inlet port (203) and a gas outlet port (204),
▪ an exhaust valve (205) configured to allow gas to escape to the atmosphere via an exhaust port (207) during each expiratory phase of the patient and to prevent gas from escaping to the atmosphere during each inspiratory phase of the patient, and
▪ a gas non-return device (208) arranged in the internal gas circuit (202), and
- a gas delivery line (300) comprising a flexible conduit having a length of between 0.5 and 10 metres, fluidically connecting, via the gas supply port (106), the internal gas passage (102) of the gas admission module (100) to the internal gas circuit (202) of the ventilation module (200), via the gas inlet port (204).

2. Device according to Claim 1, **characterized in that** the gas reservoir (107) has an internal volume of at least 20 1.

3. Device according to one of the preceding claims, **characterized in that** the gas reservoir (107) has an internal volume of less than or equal to 50 1, preferably less than or equal to 40 1.

4. Device according to Claim 1, **characterized in that** the gas admission module (100) comprises a venturi inlet (103) configured to be fluidically connected to a venturi device (133).

5. Device according to one of the preceding claims, **characterized in that** the gas admission module (100) comprises a module body (101) through which the internal gas passage (102) extends, the venturi inlet (103) and/or the gas reservoir (107) being fluidically connected to the internal gas passage (102) of the module body (101) of the gas admission module (100).

6. Device according to Claim 1, **characterized in that** the gas non-return device (208) of the ventilation module (200) comprises a one-way valve.

7. Device according to Claim 1, **characterized in that** the exhaust valve (205) comprises an internal compartment (206), a closure element (213) cooperating with a valve seat (214), an elastic means (207) arranged in the internal compartment (206) and acting on the closure element (213) so as to push it towards the valve seat (214), and an exhaust port (211) fluidically connected to the atmosphere.

8. Device according to Claim 6, **characterized in that** the exhaust valve (205) comprises:
- a PEP adjustment system (218) configured to allow a user to adjust a level of positive expiratory pressure (PEP) comprised between 0 and 30 cmH₂O, by modulating the elastic force that the elastic means (207) exerts on the closure element (213) in order to push it against the valve seat (214), or
- a non-adjustable PEP system configured to provide a fixed positive expiratory pressure (PEP) of between 0 and 30 cmH₂0.

9. Device according to Claim 1, **characterized in that** the ventilation module (200) comprises means of connection (210) to a breathing interface (250), these means being arranged at the gas outlet orifice (204) .

10. Device according to Claim 3, **characterized in that** a venturi device (133) is connected to the venturi inlet (103) of the gas admission module (100).

11. Device according to one of the preceding claims, **characterized in that**:
- a breathing interface (250) is fluidically connected to the connection means (210) of the ventilation module (200),
- an oxygen source (400) is fluidically connected to the oxygen injection port (105) of the gas admission module (100), and/or
- the gas delivery line (300) comprises a flexible tubing.

12. Device according to Claim 4, **characterized in that** the venturi inlet (103) is configured to additionally form the oxygen injection port (105), so as to allow fluidic connection either of an oxygen source or of a venturi device (133).

13. Device according to Claim 1, **characterized in that** the flexible conduit has a length of between 1 and 5 metres.

14. Device according to Claim 1, **characterized in that** the gas delivery line (300) is mechanically fixed by an upstream end to the gas admission module (100) and by a downstream end to the ventilation module (200).

15. Device according to one of Claims 1 to 3, **characterized in that** the gas reservoir (107) is made of polymer and deformable.
